# EUROPEAN PATENT SPECIFICATION

(11) **EP 4 294 308 B1**
(45) Date of publication and mention of the grant of the patent: **22.10.2025**
(21) Application number: 22709061.0
(22) Date of filing: 14.02.2022
(51) Int. Cl.: G16H 40/67, A61B 90/50, A61B 34/20, G16H 50/50, A61B 90/00, G16H 50/20, G16H 30/40, G16H 20/40, A61B 34/30, A61B 34/10, A61B 17/00

(54) **SYSTEMS AND DEVICES FOR TOOL SKIVE AVOIDANCE**
SYSTEME UND VORRICHTUNGEN ZUR VERMEIDUNG VON WERKZEUGSPRÜNGEN
SYSTÈMES ET DISPOSITIFS POUR ÉVITER LES CHUTES D'OUTILS

(30) Priority: 18.02.2021 US 202117179168
(43) Date of publication of application: 27.12.2023
(73) Proprietor: Mazor Robotics Ltd., 3079830 Caesarea (IL)
(72) Inventor: JUNIO, Dany, 3079830 Caesarea (IL); OFER, Nir, 3079830 Caesarea (IL); SANDELSON, Adi, 3079830 Caesarea (IL); SCHWARTZ, Yair S., 3079830 Caesarea (IL); GRIMBERG, Gillan M., 3079830 Caesarea (IL); DORI, Nimrod, 3079830 Caesarea (IL); SVIRI, Maor, 3079830 Caesarea (IL); KERET, Amir, 3079830 Caesarea (IL); SEEMANN, Ziv, 3079830 Caesarea (IL)
(74) Representative: Viering, Jentschura & Partner mbB Patent- und Rechtsanwälte
(86) International application number: PCT/IL2022/050172
(87) International publication number: WO 2022/175939

(56) References cited:
- WO-A1-2009/092164
- US-A1- 2011 306 985
- US-A1- 2018 085 172
- US-A1- 2020 289 133

## Description

### FIELD

The present technology generally relates to ensuring the safety and accuracy of surgical procedures, and relates more particularly to avoiding tool skive.

### BACKGROUND

Surgical procedures may involve using one or more surgical tools to drill or cut into an anatomical element at a target location. Skiving refers to the undesired slippage of a tool (e.g., along an anatomical surface) out of a target location during surgery. Skiving may occur in manual, robot-assisted, and autonomous surgical procedures.

US 2018/085172 A1 discloses a system and a method for estimating the spatial position (pose) of a tool within an object, particularly in the form of a human bone that is to be machined with said tool, comprising: a sensor being designed to be coupled to said tool, which sensor is further designed to generate an sensor output signal upon machining of said object along an actual trajectory, which sensor output signal depends on a material property of said object along said actual trajectory, an analyzing means being designed to determine a correlation between said sensor output signal upon machining said object and a plurality of pre-determined candidate output signals, each candidate output signal being generated in beforehand using said material property along an associated model trajectory in a model of said object, and wherein said analyzing means is designed to estimate a spatial position of the tool within the object using the model trajectory whose associated candidate output signal has the highest correlation with the sensor output signal, for instance.

Further systems and methods for surgical guidance as well as tool position estimation and detection are disclosed in WO 2009/092164 A1 disclosing surgical system is for use with a surgical tool and a tissue characteristic sensor associated with the surgical tool, US 2011/306985 A1 disclosing a surgical assistance system for operating on biological tissue using a surgical tool attached to an arm of an automatically-controlled surgical instrument so that an optimal feed rate of the tool is calculated and outputted to the surgical instrument, and US 2020/289133 A1 disclosing robotic manipulator supports and moves the cutting bur and one or more controllers analyze measurements from sensors and, in response, control the robotic manipulator and/or the cutting bur for purposes such as landmark detection to determine entry point, avoiding tool skiving at entry point, and avoidance of cortical wall breach during cannulation.

### SUMMARY

In view of the above, the present invention defines a system for skive avoidance as set out in claim 1.

Accordingly, a system for skive avoidance, wherein skiving refers to an undesired slippage of a surgical tool out of a target location during surgery, in accordance with claim 1 comprises: a sensor configured to measure a force exerted on a surgical tool; at least one processor; and a memory. The memory stores instructions for execution by the at least one processor that, when executed, cause the at least one processor to: project a tool trajectory onto a three-dimensional (3D) model of bone tissue; and estimate an expected normal force direction and magnitude upon contact of the surgical tool with the bone tissue, and predict a skive probability based on the expected normal force direction and magnitude.

Further advantageous embodiments are defined in the dependent claims and may be as follows.

Any of the aspects herein, wherein the memory stores additional instructions for execution by the at least one processor that, when executed, cause the at least one processor to: receive information from the sensor corresponding to a detected normal force magnitude in the expected force direction; compare the detected normal force magnitude to the expected normal force magnitude; and generate an alert when the detected normal force magnitude exceeds the expected normal force magnitude.

Any of the aspects herein, wherein the memory stores additional instructions for execution by the at least one processor that, when executed, cause the at least one processor to: receive a CT scan; and generate the 3D model based on the CT scan.

Any of the aspects herein, wherein predicting the skive probability is further based on information about the surgical tool.

Any of the aspects herein, wherein the sensor is configured to measure the force exerted on the surgical tool by measuring at least one force exerted on a robotic arm supporting the surgical tool.

Any of the aspects herein, wherein the memory stores additional instructions for execution by the at least one processor that, when executed, cause the at least one processor to: generate a torque measurement based on information about a length of the surgical tool and detected force information received from the sensor.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2A is a flowchart according to at least one embodiment of the present disclosure;
Fig. 2B is a flowchart according to at least one embodiment of the present disclosure;
Fig. 3 depicts a planned screw trajectory projected onto an anatomical model according to at least one embodiment of the present disclosure;
Fig. 4 is a flowchart according to at least one embodiment of the present disclosure;
Fig. 5A is a sample first image captured by an imaging device according to at least one embodiment of the present disclosure;
Fig. 5B is a sample second image captured by an imaging device according to at least one embodiment of the present disclosure;
Fig. 5C is an alternative sample second image captured by an imaging device according to at least one embodiment of the present disclosure;
Fig. 6 is a flowchart according to at least one embodiment of the present disclosure;
Fig. 7A is a sample first image captured by an imaging device according to at least one embodiment of the present disclosure;
Fig. 7B is a sample second image captured by an imaging device according to at least one embodiment of the present disclosure;
Fig. 7C is an alternative sample second image captured by an imaging device according to at least one embodiment of the present disclosure; and
Fig. 8 is a flowchart according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

Skiving is a problem that occurs when the surgeon's tool slides out of a designated operation area (e.g., during contact with bone tissue), and can result in a surgical procedure being conducted in an unplanned location and/or orientation. This, in turn, may lead to inaccurate implant placement and may harm the patient. During a spinal surgery, for example, a surgeon may use a drill bit to drill a hole in a vertebra for placement of a pedicle screw therein. Skiving of the drill bit may result in the hole being drilled in the wrong position and/or orientation, thus leading to implantation of the pedicle screw outside of a designated pose.

Skiving may result from poor planning. Ideally, tool trajectories are planned so that once the tool contacts the bone tissue, the bone geometry is perpendicular to the tool trajectory. If planned poorly, and the tool contacts the bone in a critical angle, large lateral forces may be generated, causing the tool to slip laterally. In some instances, however, a tool trajectory that is free of risk of skiving may not be feasible.

Skiving may also result from excessive force-e.g., pushing too hard on a tool or causing the tool to rotate when it is not docked properly to the bone may generate excessive forces that cause the tool to dock off-trajectory.

One of the challenges in guided robotic surgery in particular, but also in manual surgeries, is to be able to detect and notify a user when skiving occurs, thus giving the user the necessary feedback to enable the user to make any needed corrections.

At least some embodiments of the present disclosure enable skiving to be detected so that the surgeon can be alerted and can reposition the tool in the right trajectory before commencing or continuing a surgical procedure (e.g., so that a surgeon can reposition a drill bit in the corrected trajectory and/or at the planned drilling location before drilling a hole in a vertebra and implanting a pedicle screw therein).

According to at least some embodiments of the present disclosure, a small camera may be positioned on or near a tool to be used by a surgeon for a surgical procedure. The camera is oriented parallel to the tool trajectory when positioned on the tool, and either parallel or at an angle to the tool trajectory when positioned near the tool. When the camera is positioned near the tool, the camera is mounted (whether directly or indirectly) to an object supporting the tool, such that movement of the tool other than along the predetermined tool trajectory will cause movement of the camera. Whether the camera is mounted on or near the tool, the camera points toward the target location for the surgical procedure (e.g., a target drilling location). An image captured by the camera may be annotated by placing a digital marker therein to identify the target location. As the tool is brought into contact with the patient's anatomy and the procedure commences, one or more additional images may be captured with camera.

Where the camera is positioned on the tool and parallel to the tool trajectory, these one or more additional images (which may or may not be annotated to show the digital marker) may be compared to the initial image to determine whether the field of view has simply "zoomed in" (e.g., due to the tool and camera being moved closer to the target contact location), or has instead shifted to one side or another (thus indicating that skiving has occurred). Where the camera is positioned near the tool, these one or more additional images may be compared to the initial image to determine whether the field of view has remained constant, or has instead moved (thus indicating that skiving has occurred). Regardless of the camera position, image processing may be used to detect whether the tool has moved out of the predetermined trajectory relevant to the vertebra itself. In some embodiments, a detected movement must exceed a predetermined threshold in order for the surgeon or other user to be notified to reposition the tool.

Notably, the foregoing embodiments enable detection of movement of the patient's anatomy relative to the predetermined trajectory as well as detection of movement of the surgical tool relative to the predetermined trajectory.

Cameras and other imaging devices used in the foregoing embodiments may be positioned and/or oriented inside a tool, or may be attached to the tool, to a tool guide, or to an arm guide. The camera may be a stand-alone camera or may have a wired or wireless connection to a navigation system, a robotic system, or another system. The camera may be a borescope camera. Also in some embodiments, a physical marker may be inserted into a patient's anatomy and attached at the target location, rather than annotating one or more camera images with a digital marker.

According to at least some embodiments of the present disclosure, a combined hardware and software solution is used to address the challenge of skiving detection and avoidance. From the hardware side, an external force/torque sensor may be attached to a robotic arm to indicate in real time the exerted forces and torques the robotic arm senses during use in surgery. The forces and torques may alternative be interpolated from reading any one of the robotic arm control parameters, such as currents and/or positional error, and/or may be determined from another sensor reading. Forces may be measured on any one or more of the tip of the robot (where the tool is placed), within the robotic joints, or at the robot-patient interface.

On the software side, known bone anatomy (extrapolated, for example, from a CT or MRI scan) may be used to estimate the direction in which skive is most likely to occur (e.g., normal to the bone surface). Additionally, a navigation system may be used to understand the exact tool location in relation to the robotic system and/or the anatomy, to track any unwanted motion, and/or to better interpolate the generated forces. Combining the hardware and software aspects and correlating the predicted skive direction with the measured force/torque direction, a threshold may be determined such that when the threshold is passed, a skive is likely to occur. In some embodiments, historical information about the probability of a specific tool to cause skive may also be taken into account and used to determine when skive is likely.

According to some embodiments of the present disclosure, a CT scan or other three-dimensional (3D) image may be used to build a 3D model representing a patient's bone tissue. A planned implant or tool trajectory may then be projected onto the 3D model, and a normal direction (e.g., a direction of expected force generated upon contact of the tool with the bone tissue) may be estimated. The estimating may be based, for example, on a determination of a bone surface gradient at the point of contact, and on an angle between the bone surface gradient and the planned trajectory. Forces (both magnitude and direction) monitored in real-time during tool insertion may be compared with prior knowledge of the expected normal direction (e.g., the estimated normal direction) to determine whether an excess force is measured in the estimated normal direction, in which case a user may be alerted.

Additional inputs may be used to increase the accuracy of a skive detection method as described herein. Navigation or other tracking technology may be used to estimate the length of a tool and to calculate a torque (e.g., by multiplying a measured or otherwise detected or calculated force by the moment arm), which may then be used alongside force measurements to predict a skive probability and/or to detect tool skive. Additionally or alternatively, a preoperative plan may be used to estimate tool length, based on a planned position of a tool guide (whether held by a robotic arm or otherwise) as well as the planned location at which the tool is expected to meet or contact the bone tissue. This distance can be used for accurate torque to force (or vice versa) conversions and skive probability considerations. Information about the tool type, including the tool flexibility and tool geometry, may also be a useful input for a skive probability calculation and/or skive detection. For some tools, low forces can result in flexibility that may yield skive. Additionally, the diameter of a tool tip, for example, can be considered in a skive probability analysis: a cannula or drill guide has a known diameter that will make point-contact on its outer edge, which may not correspond to the centerline of the tool. This may contribute to higher skive probability, which in turn may affect whether a decision is made to alert the user regarding the likelihood of tool skive based on a particular preoperative plan.

Further still, navigation may be used to estimate skive direction (if skive has occurred) which in turn may be used as an input to determine whether to alert the user. As noted above, robotic arm control parameters (e.g., consumed currents, encoder readings) may indicate that excessive force is being used and that a skive alert is required.

According to at least some embodiments of the present disclosure, a patient-anatomy specific skiving threshold may be established. The skiving threshold may be a three-dimensional threshold that varies in each vector. One of the problems associated with skiving is defining what magnitude of displacement of the tool from the target contact location is considered to be skiving. Embodiments of the present disclosure analyze the information from a CT scan or other preoperative image, as well as a preoperative plan, and calculate a distance and direction between a planned implant (or other procedure-specific intervention) and critical organs or other anatomical elements. For example, in the spinal surgery context, embodiments of the present disclosure may be used to calculate a distance and direction between a planned pedicle screw and a spinal cord. These calculations enable determination of a 3D skiving threshold, which may vary depending on the direction in which critical anatomical elements are located. For example, if a critical anatomical element is closer in the anterior direction than in the posterior direction, a tool may need to slip or be otherwise displaced by 3 mm in a posterior direction in order for the displacement or slippage to be considered skiving, but by only 1 mm in the anterior direction.

Once the skiving threshold has been determined, expected and/or actual forces and/or torques at the surgical tool-patient interface may be calculated or measured and compared to the calculated threshold, and a surgeon or other user may be alerted if the expected and/or actual forces and/or torques exceed the calculated threshold.

Embodiments of the present disclosure provide technical solutions to one or more of the problems of (1) detecting tool skive; (2) predicting a probability of tool skive; (3) determining appropriate thresholds for acceptable and/or unacceptable amounts of tool skive; (4) detecting anatomic movement relative to a surgical tool; (5) preventing false alarms of skiving, particularly in directions where skiving is less problematic; (6) alerting a doctor or other user if a preoperative plan has a high likelihood of skiving and/or if the nature of the planned procedure will prevent effective skiving detection; and (7) increasing the accuracy of skiving detection.

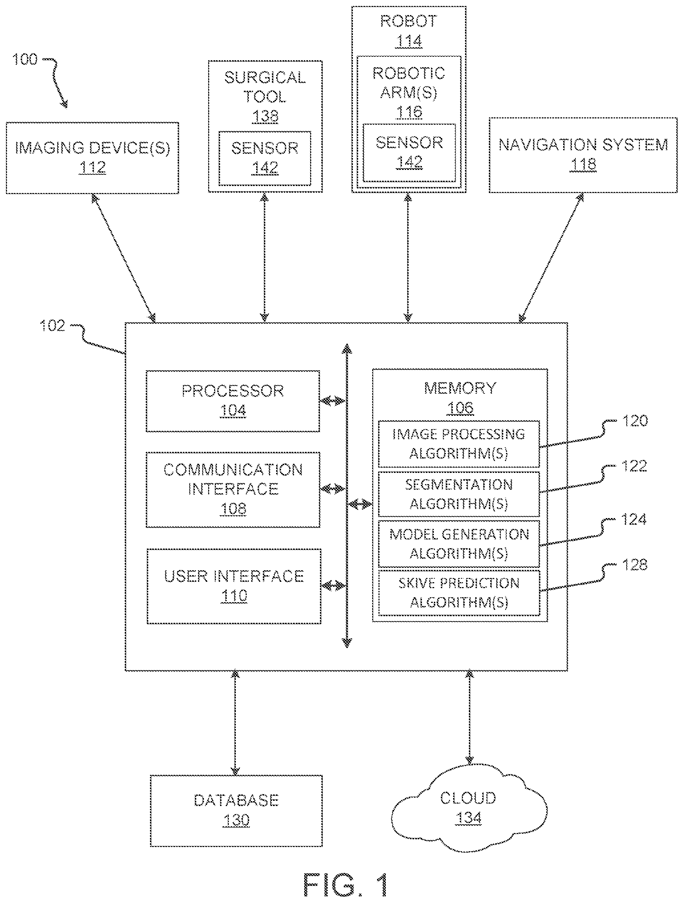

Turning first to Fig. 1, a block diagram of a system 100 according to at least one embodiment of the present disclosure is shown. The system 100 may be used to determine a skive probability based on a surgical plan, to detect skiving when during a surgical procedure, to alert a surgeon or other user of actual or potential skiving, and/or to carry out one or more other aspects of one or more of the methods disclosed herein. The system 100 comprises a computing device 102, one or more imaging devices 112, a robot 114, a navigation system 118, a database 130, a cloud or other network 134, and a surgical tool 138. Systems according to other embodiments of the present disclosure may comprise more or fewer components than the system 100. For example, the system 100 may not include the imaging device 112, the robot 114, the navigation system 118, one or more components of the computing device 102, the database 130, the cloud 134, and/or the surgical tool 138.

The computing device 102 comprises a processor 104, a memory 106, a communication interface 108, and a user interface 110. Computing devices according to other embodiments of the present disclosure may comprise more or fewer components than the computing device 102.

The processor 104 of the computing device 102 may be any processor described herein or any similar processor. The processor 104 may be configured to execute instructions stored in the memory 106, which instructions may cause the processor 104 to carry out one or more computing steps utilizing or based on data received from the imaging device 112, the robot 114, the navigation system 118, the database 130, the cloud 134, and/or the surgical tool 138.

The memory 106 may be or comprise RAM, DRAM, SDRAM, other solid-state memory, any memory described herein, or any other tangible, non-transitory memory for storing computer-readable data and/or instructions. The memory 106 may store information or data useful for completing, for example, any step of the methods 200, 400, 600, and/or 800 described herein, or of any other methods. The memory 106 may store, for example, one or more image processing algorithms 120, one or more segmentation algorithms 122, one or more model generation algorithms 124, and/or one or more skive prediction algorithms 128. Such instructions or algorithms may, in some embodiments, be organized into one or more applications, modules, packages, layers, or engines. The algorithms and/or instructions may cause the processor 104 to manipulate data stored in the memory 106 and/or received from or via the imaging device 112, the robot 114, the database 130, the cloud 134, and/or the surgical tool 138.

The computing device 102 may also comprise a communication interface 108. The communication interface 108 may be used for receiving image data or other information from an external source (such as the imaging device 112, the robot 114, the navigation system 118, the database 130, the cloud 134, the surgical tool 138, and/or any other system or component not part of the system 100), and/or for transmitting instructions, images, or other information to an external system or device (e.g., another computing device 102, the imaging device 112, the robot 114, the navigation system 118, the database 130, the cloud 134, the surgical tool 138, and/or any other system or component not part of the system 100). The communication interface 108 may comprise one or more wired interfaces (e.g., a USB port, an ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 108 may be useful for enabling the device 102 to communicate with one or more other processors 104 or computing devices 102, whether to reduce the time needed to accomplish a computing-intensive task or for any other reason.

The computing device 102 may also comprise one or more user interfaces 110. The user interface 110 may be or comprise a keyboard, mouse, trackball, monitor, television, screen, touchscreen, and/or any other device for receiving information from a user and/or for providing information to a user. The user interface 110 may be used, for example, to receive a user selection or other user input regarding any step of any method described herein. Notwithstanding the foregoing, any required input for any step of any method described herein may be generated automatically by the system 100 (e.g., by the processor 104 or another component of the system 100) or received by the system 100 from a source external to the system 100. In some embodiments, the user interface 110 may be useful to allow a surgeon or other user to modify instructions to be executed by the processor 104 according to one or more embodiments of the present disclosure, and/or to modify or adjust a setting of other information displayed on the user interface 110 or corresponding thereto.

Although the user interface 110 is shown as part of the computing device 102, in some embodiments, the computing device 102 may utilize a user interface 110 that is housed separately from one or more remaining components of the computing device 102. In some embodiments, the user interface 110 may be located proximate one or more other components of the computing device 102, while in other embodiments, the user interface 110 may be located remotely from one or more other components of the computing device 102.

The imaging device 112 may be operable to image anatomical feature(s) (e.g., a bone, veins, tissue, etc.) and/or other aspects of patient anatomy to yield image data (e.g., image data depicting or corresponding to a bone, veins, tissue, etc.). "Image data" as used herein refers to the data generated or captured by an imaging device 112, including in a machine-readable form, a graphical/visual form, and in any other form. In various examples, the image data may comprise data corresponding to an anatomical feature of a patient, or to a portion thereof. The image data may be or comprise a preoperative image, an intraoperative image, a postoperative image, or an image taken independently of any surgical procedure. In some embodiments, a first imaging device 112 may be used to obtain first image data (e.g., a first image) at a first time, and a second imaging device 112 may be used to obtain second image data (e.g., a second image) at a second time after the first time. The imaging device 112 may be capable of taking a 2D image or a 3D image to yield the image data. The imaging device 112 may be or comprise, for example, an ultrasound scanner (which may comprise, for example, a physically separate transducer and receiver, or a single ultrasound transceiver), an O-arm, a C-arm, a G-arm, or any other device utilizing X-ray-based imaging (e.g., a fluoroscope, a CT scanner, or other X-ray machine), a magnetic resonance imaging (MRI) scanner, an optical coherence tomography (OCT) scanner, an endoscope, a microscope, an optical camera, a thermographic camera (e.g., an infrared camera), a radar system (which may comprise, for example, a transmitter, a receiver, a processor, and one or more antennae), or any other imaging device 112 suitable for obtaining images of an anatomical feature of a patient. The imaging device 112 may be contained entirely within a single housing or may comprise a transmitter/emitter and a receiver/detector that are in separate housings or are otherwise physically separated.

In some embodiments, the imaging device 112 may comprise more than one imaging device 112. For example, a first imaging device may provide first image data and/or a first image, and a second imaging device may provide second image data and/or a second image. In still other embodiments, the same imaging device may be used to provide both the first image data and the second image data, and/or any other image data described herein. The imaging device 112 may be operable to generate a stream of image data. For example, the imaging device 112 may be configured to operate with an open shutter, or with a shutter that continuously alternates between open and shut so as to capture successive images. For purposes of the present disclosure, unless specified otherwise, image data may be considered to be continuous and/or provided as an image data stream if the image data represents two or more frames per second.

The robot 114 may be any surgical robot or surgical robotic system. The robot 114 may be or comprise, for example, the Mazor X^{™} Stealth Edition robotic guidance system. The robot 114 may be configured to position the imaging device 112 at one or more precise position(s) and orientation(s), and/or to return the imaging device 112 to the same position(s) and orientation(s) at a later point in time. The robot 114 may additionally or alternatively be configured to manipulate a surgical tool (whether based on guidance from the navigation system 118 or not) to accomplish or to assist with a surgical task. In some embodiments, the robot 114 may be configured to hold and/or manipulate an anatomical element during or in connection with a surgical procedure.

The robot 114 may comprise one or more robotic arms 116. In some embodiments, the robotic arm 116 may comprise a first robotic arm and a second robotic arm, though the robot 114 may comprise more than two robotic arms. In some embodiments, one or more of the robotic arms 116 may be used to hold and/or maneuver the imaging device 112. In embodiments where the imaging device 112 comprises two or more physically separate components (e.g., a transmitter and receiver), one robotic arm 116 may hold one such component, and another robotic arm 116 may hold another such component. Each robotic arm 116 may be positionable independently of the other robotic arm. The robotic arms may be controlled in a single, shared coordinate space, or in separate coordinate spaces.

Each robotic arm 116 may comprise one or more sensors 142. The one or more sensors 142 enable the processor 104 (or a processor of the robot 114) to determine a precise pose in space of the robotic arm (as well as any object or element held by or secured to the robotic arm), and/or to determine a force and/or torque exerted by or on the robotic arm 116. For example, where the robotic arm 116 comprises a hinge joint, a sensor 142 may detect an angular position of a robotic arm member extending from the hinge joint relative to an axis of the hinge joint. Where the robotic arm 116 comprises a rotary joint, the sensor 142 may detect an angular position of a robotic arm member relative to the axis that extends through the robotic arm member and the rotary joint. Each sensor 142 may be, for example, a rotary encoder, a linear encoder, or an incremental encoder. Each sensor may also be or include a force sensor, a pressure sensor, a torque sensor, and/or a current sensor. Data from the sensors 142 may be provided, for example, to a processor of the robot 114, to the processor 104 of the computing device 102, and/or to the navigation system 118. The data may be used in connection with one or more aspects of one or more methods disclosed herein.

The robot 114, together with the robotic arm 116, may have, for example, one, two, three, four, five, six, seven, or more degrees of freedom. Further, the robotic arm 116 may be positioned or positionable in any pose, plane, and/or focal point. The pose includes a position and an orientation. As a result, an imaging device 112, surgical tool 138, or other object held by the robot 114 (or, more specifically, by the robotic arm 116) may be precisely positionable in one or more needed and specific positions and orientations.

In some embodiments, reference markers (i.e., navigation markers) may be placed on the robot 114 (including, e.g., on the robotic arm 116), the imaging device 112, or any other object in the surgical space. The reference markers may be tracked by the navigation system 118, and the results of the tracking may be used by the robot 114 and/or by an operator of the system 100 or any component thereof. In some embodiments, the navigation system 118 can be used to track other components of the system (e.g., imaging device 112) and the system can operate without the use of the robot 114 (e.g., with the surgeon manually manipulating the imaging device 112 and/or one or more surgical tools 138, based on information and/or instructions generated by the navigation system 118, for example).

The navigation system 118 may provide navigation for a surgeon and/or a surgical robot during an operation. The navigation system 118 may be any now-known or future-developed navigation system, including, for example, the Medtronic StealthStation^{™} S8 surgical navigation system or any successor thereof. The navigation system 118 may include one or more cameras or other sensor(s) for tracking one or more reference markers, navigated trackers, or other objects within the operating room or other room in which some or all of the system 100 is located. The one or more cameras may be optical cameras, infrared cameras, or other cameras. In some embodiments, the navigation system may comprise one or more electromagnetic sensors. In various embodiments, the navigation system 118 may be used to track a position and orientation (i.e., pose) of the imaging device 112, the robot 114 and/or robotic arm 116, and/or one or more surgical tools 138 (or, more particularly, to track a pose of a navigated tracker attached, directly or indirectly, in fixed relation to the one or more of the foregoing). The navigation system 118 may include a display for displaying one or more images from an external source (e.g., the computing device 102, imaging device 112, or other source) or for displaying an image and/or video stream from the one or more cameras or other sensors of the navigation system 118. In some embodiments, the system 100 can operate without the use of the navigation system 118. The navigation system 118 may be configured to provide guidance to a surgeon or other user of the system 100 or a component thereof, to the robot 114, or to any other element of the system 100 regarding, for example, a pose of one or more anatomical elements, whether or not a tool is in the proper trajectory, and/or how to move a tool into the proper trajectory to carry out a surgical task according to a preoperative or other surgical plan.

The surgical tool 138 may be or comprise any surgical tool for which skiving is or may be a concern during use, including, for example, a drill. The surgical tool 138 may be configured to be supported and operated manually; to be supported robotically but operated manually; and/or to be supported and operated robotically. The surgical tool 138 may comprise one or more sensors 142, which were described above. Such sensors 142 may detect, for example, one or more forces or torques exerted on or by the surgical tool 138; current consumed by the surgical tool 138; and/or a position of one or more components of the surgical tool 138 relative to one or more other components of the surgical tool 138.

The system 100 or similar systems may be used, for example, to carry out one or more aspects of any of the methods 200, 400, 600, and/or 800 described herein. The system 100 or similar systems may also be used for other purposes.

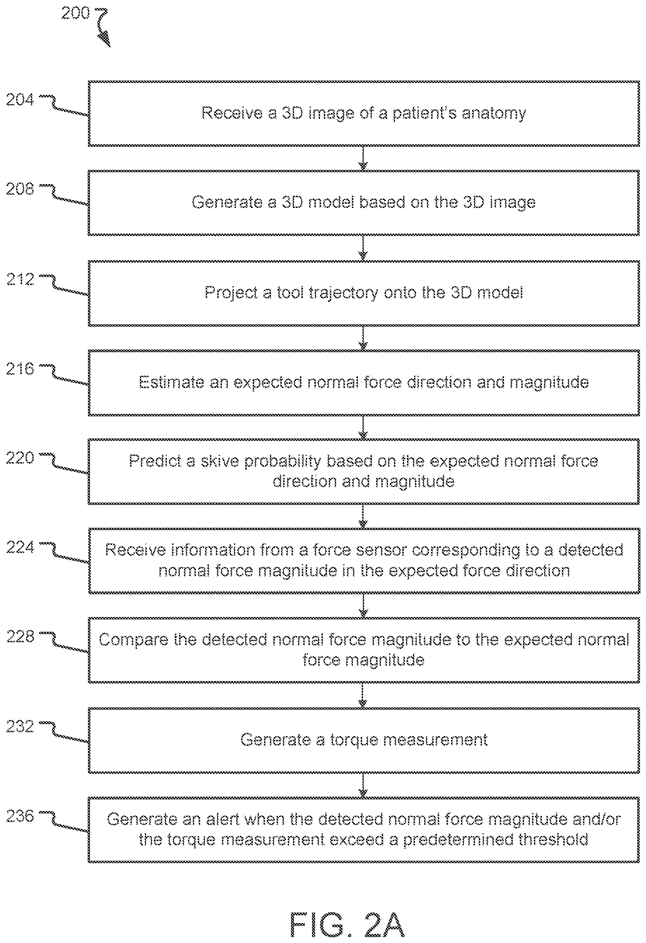

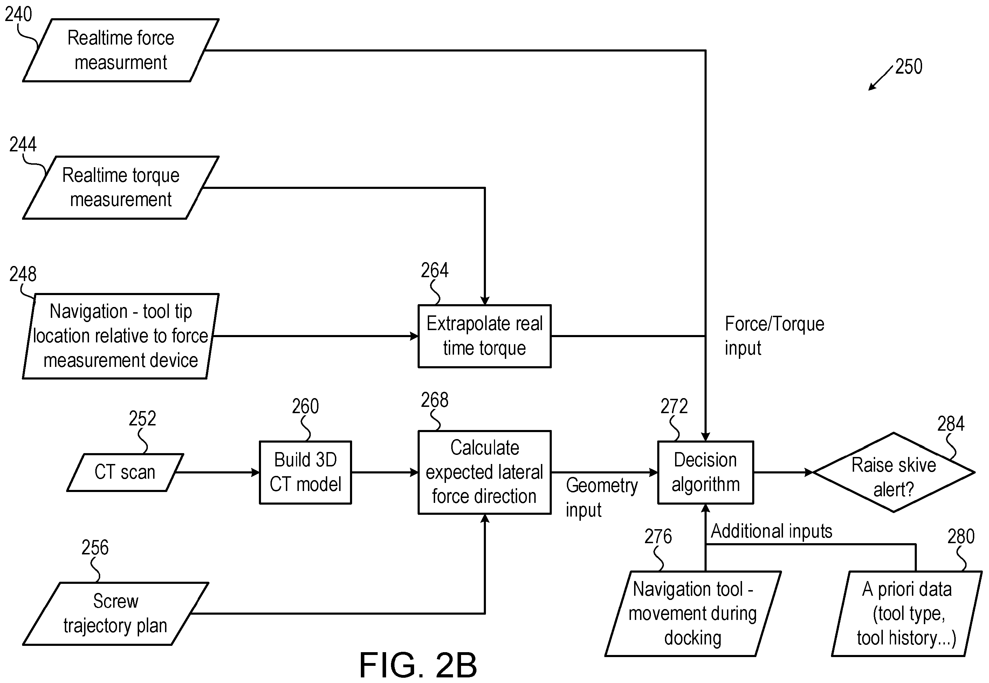

Fig. 2 depicts a method 200 that may be used, for example, to predict a skive probability, to detect skiving, and/or to alert a surgeon or other user regarding a predicted skive probability and/or detected skiving.

The method 200 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 200. The at least one processor may perform the method 200 by executing instructions stored in a memory such as the memory 106. The instructions may correspond to one or more steps of the method 200 described below. The instructions may cause the processor to execute one or more algorithms, such as an image processing algorithm 120, a segmentation algorithm 122, a model generation algorithm 124, and/or a skive prediction algorithm 128.

The method 200 comprises receiving a 3D image of a patient's anatomy (step 204). The 3D image may be a computed tomography (CT) image, a magnetic resonance imaging (MRI) image, or any other 3D image. The patient is a patient on whom a surgical procedure will be conducted, and the patient's anatomy depicted in the image is at least that portion of the patient's anatomy that is relevant to the surgical procedure. For example, if the patient is to undergo a spinal fusion procedure that involves implanting pedicles screws into a plurality of vertebrae of the patient's spine, and connecting the implanted pedicle screws with a rod, then the 3D image is an image of at least the patient's spine and surrounding anatomy. In some embodiments, the 3D image may depict only a single anatomical element of a patient, or only a portion of a single anatomical element of the patient (where the portion includes a target contact location at which a surgical tool such as a surgical tool 138 is expected to make contact with the anatomical element). The 3D image may depict only bony tissue, or the 3D image may depict both hard tissue and soft tissue. Where the surgical procedure will not involve contact between a surgical tool and one or more bony anatomical elements, but skiving is still a concern, the 3D image may depict only soft tissue.

The 3D image may be received from and/or via a memory 106, a communication interface 108, a user interface 110, an imaging device 112, a database 130, or a network such as the cloud 134. The 3D image may be received immediately after or upon generation of the 3D image, or the 3D image may be an image previously captured and stored.

In some embodiments, the step 204 may comprise causing an imaging device (such as an imaging device 112) to capture a 3D image of the patient's anatomy.

The method 200 also comprises generating a 3D model based on the 3D image (step 208). The 3D model may be generated using a model generation algorithm 124. The model may depict only a single anatomical element that a surgical tool (e.g., a surgical tool 138) will contact during the surgical procedure, or more than one anatomical element. The 3D model may depict, for example, a contour of a bony anatomical element at a target contact location, where a surgical tool is expected to contact the bony anatomical element. The 3D model may be made using only the 3D image as input, or the 3D model may be generated using a plurality of inputs, including the 3D image. For example, an anatomical atlas, one or more 2D images, medical information about the patient in question; and/or other information may be used as inputs in the generation of the 3D model.

The method 200 also comprises projecting a tool trajectory onto the 3D model (step 212). The tool trajectory may be input via a user interface such as the user interface 110, and/or may be extracted or otherwise obtained from a preoperative plan corresponding to a surgical procedure to be performed on the patient. The step 212 may yield, for example, a visual depiction of the tool trajectory within the 3D model, such that a precise expected point where the surgical tool will contact the bone (referred to herein as a "target contact point" or "target contact location") may be seen and/or determined. Any registration or other correlation process needed to ensure the tool trajectory is accurately projected onto the 3D model may be conducted as part of the step 212.

The method 200 also comprises estimating an expected normal force direction and magnitude (step 216). The estimating may comprise defining a plane that passes through the target contact point and is tangent to the surface of the anatomical element (as depicted in the 3D model) at that point. The expected normal force direction may then be defined as a direction perpendicular to the tangent plane, extending away from the anatomical element at the target contact point. Other methods for defining the expected normal force direction may alternatively be used.

Estimating the expected normal force magnitude may be based on multiple inputs, including, for example, information about the anatomical element into which the tool trajectory extends; information about the patient (e.g., age, weight, bone mineral density); information about the surgical tool that will be placed in contact with the anatomical element (e.g., flexibility, shape, weight, operating parameters); and/or historical information about forces exerted on the surgical tool in question or similar tools during the same or similar surgical procedures.

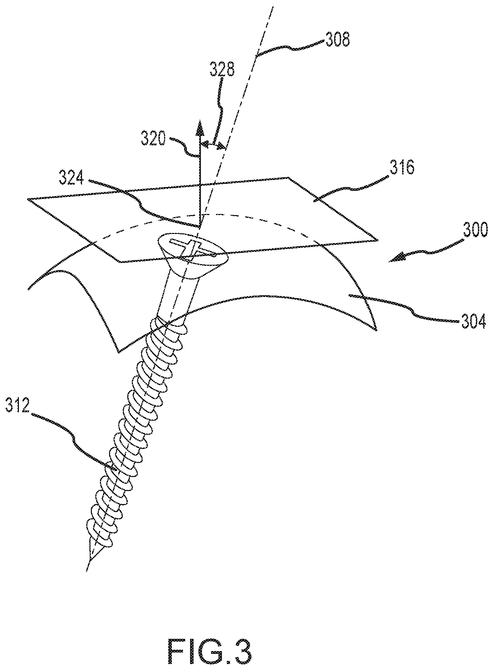

Turning briefly to Fig. 3, a 3D model 300 of a vertebra 304 is depicted. A tool trajectory 308 (to be used by a drill for drilling a hole in the vertebra 304 that will accept a planned pedicle screw implant 312) has been projected onto the 3D model 300. A tangent plane 316, which passes through the target contact point 324 and is tangent to a surface of the vertebra 304 at the target contact point 324, has been defined. The expected normal force direction 320 is the direction perpendicular to the tangent plane 316, extending through the target contact point 324 and away from the vertebra 304.

Returning now to Fig. 2, the method 200 also comprises predicting a skive probability based on the expected normal force direction and magnitude (step 220). Skiving is less likely when the angle 328 between the normal force direction and the tool trajectory is closer to zero degrees, and increasingly more likely as the angle 328 increases (e.g., when drilling at an angle 328 that is farther away from zero). The expected normal force magnitude may also affect the probability of skiving, with different combinations of normal force magnitudes and directions yielding different probabilities of skiving. In some embodiments, a lookup table with skiving probability values indexed by expected normal force direction and expected normal force magnitude may be referenced during the step 220 to determine a predicted skive probability. In other embodiments, an algorithm such as the skive prediction algorithm 128 may be used to calculate a predicted skive probability based on the expected normal force direction and magnitude. Also in some embodiments, additional inputs, beyond the expected normal force direction and magnitude, may be used by a skive prediction algorithm 128 or any other algorithm to predict a skive probability. Such inputs may include, for example, expected, actual, and/or calculated torque measurements (e.g., torque exerted on or by the surgical tool); data from a navigation or other system useful for calculating a distance between a tip of the surgical tool and a force measurement device (e.g., for calculating torque); information about a distance between a tool guide or other tool supporting arm and the target contact point (e.g., for calculating torque, and/or calculating skive probability independent of any torque calculations); and/or one or more tool properties (e.g., tool flexibility, geometry). Any other input described herein may be used to predict a skive probability.

The method 200 also comprises receiving information from a sensor corresponding to a detected normal force magnitude in the expected force direction (step 224). The sensor may be, for example, a sensor 142. The sensor may be operably connected to the surgical tool, a robotic arm supporting the surgical tool, any other tool guide or support for the surgical tool and/or at a robot - patient interface-provided, in each case, the sensor is positioned to detect forces exerted on or by the surgical tool. Detecting a force, for purposes of the present disclosure, includes both direct detection and/or measurement of the force magnitude and/or direction, and detection and/or measurement of one or more parameters from which the force magnitude and/or direction may be calculated. Thus, for example, the sensor may be a force sensor configured to detect force directly. Alternatively, the sensor may be a current sensor configured to detect electrical current draw (e.g., by one or more motors of a robotic arm as the motors operate to maintain the robotic arm in a predetermined pose), positional error of a robotic arm (e.g., resulting from an external force being applied on the robotic arm), or any other parameters useful for indirectly calculating a normal force magnitude and/or direction.

Where the information received in the step 224 does not include an actual value for the normal force direction and/or magnitude, but instead includes information from which the actual value (or at least a substantially accurate estimate thereof) may be calculated, then the step 224 includes calculating the normal force direction and/or magnitude, as appropriate.

In some embodiments, the step 224 also includes receiving information from a sensor corresponding to a detected or otherwise measured torque exerted on or by the surgical tool.

The method 200 also comprises comparing the detected normal force magnitude to the expected normal force magnitude (step 228). The comparing may be, for example, a simple comparison to determine whether the detected normal force magnitude is larger or smaller than the expected normal force magnitude. Such a comparison may be made, for example, by subtracting the detected normal force magnitude from the expected normal force magnitude. If the result is positive, then the detected normal force magnitude is lower than the expected normal force magnitude, suggesting a decreased risk or probability of skiving. If the result is negative, then the detected normal force magnitude is higher than the expected normal force magnitude, suggesting an increased risk or probability of skiving. In some embodiments, a skiving probability calculated in real time based on the detected normal force magnitude (and/or based on the comparison of the detected normal force magnitude with the expected normal force magnitude) may be displayed or otherwise provided to a user.

In some embodiments, the comparing may utilize an algorithm. For example, the comparing may utilize an algorithm that weights one or both of the expected normal force magnitude and/or the detected normal force magnitude, and compares the resulting weighted value(s). Also in some embodiments, the comparing may utilize one or more additional inputs (in addition to the detected and expected normal force magnitudes).

Although the step 228 is described as comparing the detected normal force magnitude to the expected normal force magnitude, in some embodiments, the step 228 may comprise calculating a skive probability based on the detected normal force magnitude (without regard for the expected normal force magnitude). In such embodiments, the skive probability may be calculated in any manner described herein.

The method 200 also comprises generating a torque measurement (step 232). The torque measurement may be generated, for example, by multiplying a detected force magnitude (e.g., from the step 224) by a length of the applicable moment arm. The applicable moment arm may be, for example, a length of the tool in question between a location of the sensor used to detect the force and a tip of the tool (where the force is exerted on the tool). That length may be obtained from a memory such as the memory 106 or the database 130; looked up using a network such as the cloud 134; measured or otherwise calculated using a surgical plan defining a pose of a robotic arm or other tool guide relative to a target contact point; measured or otherwise calculated using data obtained from a navigation system; or obtained, calculated, or measured from any other source and/or using any other data.

The generated torque measurement may be used, for example, to calculate and/or update a previously calculated skive probability. The generated torque measurement may also be compared, for example, to an expected torque value (which may be calculated, for example, based on the tool length, determined as described above, and the expected normal force magnitude).

The method 200 also comprises generating an alert when the detected normal force magnitude and/or the torque measurement exceed a predetermined threshold (step 236). The predetermined threshold may be, for example, the expected normal force magnitude, and/or an expected torque measurement, as appropriate. The predetermined threshold may alternatively be calculated based on the expected normal force magnitude and/or an expected torque measurement. As yet another alternative, the predetermined threshold may be probability-based. Thus, for example, if the detected normal force results in a calculated or otherwise predicted skive probability of greater than 50%, or greater than 60%, or greater than 70%, or greater than 80%, or greater than 90%, then generation of the alert may be triggered.

The present disclosure encompasses embodiments of the method 200 that comprise more or fewer steps than those described above, and/or one or more steps that are different than the steps described above.

Fig. 2B provides a flowchart of a method 250, which may be the same as or similar to the method 200. Although the method 250 is described in the context of a surgical procedure to implant a pedicle screw in a patient's vertebra, the method 250 is applicable to any situation in which skiving may occur. The method 250 concludes with a decision step 284, in which a decision is made as to whether to raise a skive alert (e.g., whether to alert a surgeon or other user that skiving is likely or is actually occurring). The decision is made using a decision algorithm 272, which may be based on, for example, inputs related to the geometry of the patient's anatomy and the planned tool trajectory; inputs regarding actual force and/or torque during tool insertion; and one or more additional inputs. Each of these categories is discussed below.

Determination of the geometry input begins with a CT scan or other 3D image 252 (which may be received, for example, as described above in connection with the step 204 of the method 200) and a screw trajectory plan 256 (which may be received, for example, by a processor such as the processor 104, from or via a memory 106, a communication interface 108, a user interface 110, an imaging device 112, a database 130, or a network such as the cloud 134. The CT scan may be used to build a 3D model 260 of the relevant patient anatomy (as described above, for example, in connection with the step 208 of the method 200)-in this case, a vertebra. A tool or implant trajectory may then be extracted or otherwise obtained from the screw trajectory plan 256, and projected onto the 3D model (as describe above, for example, in connection with the step 212 of the method 200) so that an expected lateral force direction 268 (and, in some embodiments, an expected lateral force magnitude) may be calculated (as described above, for example, in connection with the step 216 of the method 200). The expected lateral force direction may then be input into the decision algorithm 272.

The force/torque input is obtained through real-time force measurement 240 (as described, for example, in connection with the step 224 of the method 200), through real-time torque measurement 244 (as described, for example, in connection with the step 224 of the method 200), and/or by using data 248-from a navigation system or other source regarding a position of the tool tip relative to a position of a force measurement device (e.g., a sensor 142)-to determine an extrapolated or otherwise calculated real-time torque 264 exerted on or by the surgical tool (as described above, for example, in connection with the step 232 of the method 200). The real-time force measurement and either the real-time torque measurement or the extrapolated or otherwise calculated torque measurement are then provided as inputs to the decision algorithm 272.

The additional inputs may include an input 276 from a navigation tool regarding tool movement during docking of the surgical tool (e.g., a surgical tool 138) with the anatomical element in question, and/or a priori data 280 regarding tool type, tool history, and/or any other known information relevant to detecting a skive probability (described in more detail, for example, in connection with the step 220 of the method 200).

With the inputs described above (or a subset thereof), the decision algorithm 272 may be used to determine whether to raise a skive alert. The determination may be made, and where appropriate an alert may be generated, for example, in the same manner as or in a similar manner to the step 236 of the method 200.

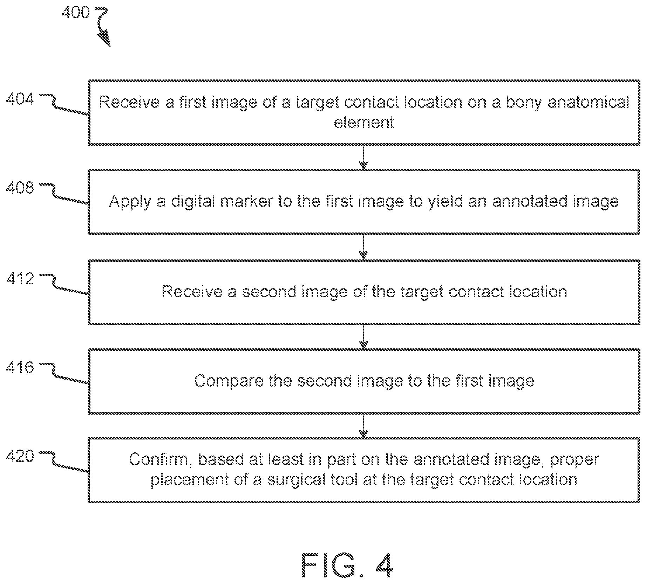

Fig. 4 depicts a method 400 that may be used, for example, to detect and avoid likely or actual skiving.

The method 400 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 400. The at least one processor may perform the method 400 by executing instructions stored in a memory such as the memory 106. The instructions may correspond to one or more steps of the method 400 described below. The instructions may cause the processor to execute one or more algorithms, such as an image processing algorithm 120, a segmentation algorithm 122, a model generation algorithm 124, and/or a skive prediction algorithm 128.

The method 400 comprises receiving a first image of a target contact location on a bony anatomical element (step 404). The first image is generated by a camera or other imaging device that is attached to a surgical tool (e.g., a surgical tool 138) that will be used to contact a bony anatomical element of a patient (e.g., a drill). The camera or other imaging device is oriented parallel to the tool trajectory. In some embodiments, the camera or other imaging device is oriented along the tool trajectory, such that a center of the camera's field of view corresponds with the target contact location. In other embodiments, the camera or other imaging device is offset from the tool trajectory, such that the target contact location is within, but not at the center of, the camera's field of view.

The camera or other imaging device may be in wired or wireless connection to a computing device (e.g., a computing device 102), a robot (e.g., a robot 114), a navigation system (e.g., a navigation system 118), or any other component of a system such as the system 100. The camera or other imaging device may be, for example, an imaging device 112.

The target contact location is a location at which the surgical tool must contact the bony anatomical element to achieve a planned result (e.g., to drill a holed in the correct pose). The bony anatomical element may a vertebra or any other bony anatomical element.

The method 400 also comprises applying a digital marker to the first image to yield an annotated image (step 408). The digital marker identifies the target contact location, and may be or comprise a digital overlay placed on the image to mark the target contact location. The digital marker may be or comprise a plurality of intersecting lines (with the point of intersection corresponding to the target location), an arrow (with the tip of the arrow corresponding to the target contact location), a circle (with the center of the circle corresponding to the target contact location), or any other suitable shape or configuration to identify the target contact location. An algorithm such as an image processing algorithm 120 may be used to apply the digital marker to the first image.

In other methods according to embodiments of the present disclosure, the first image is not annotated.

The method 400 also comprises receiving a second image of the target contact location (step 412). The second image of the target contact location is captured by the same camera or other imaging device that captured the first image. However, the second image is taken as the surgical tool is inserted into the patient, and as a result, at the time of taking the second image, the camera or other imaging device is closer to the target contact location than at the time of taking the first image. Stated differently, the second image is taken when the camera or other imaging device is a second distance from the target contact location, while the first image is taken when the camera or other imaging device is at a first distance from the target contact location, with the first distance being greater than the second distance.

The second image may, in some embodiments, be annotated to include a digital marker just as the first image.

The method 400 also comprises comparing the second image to the first image (step 416). Because the camera or other imaging device used to obtain the first and second images is attached to the surgical tool and oriented along the tool trajectory, proper placement of the tool along the tool trajectory should result in the second image being centered at the same point of the bony anatomical element as the first image. In other words, if no undesired motion of the surgical tool (to which the camera or other imaging device is attached) or the patient occurred, then the second image should simply be a magnified or zoomed-in image of the center of the first image. Thus, if the target contact location was at the center of the first image, then the target contact location should also be at the center of the second image if the tool has been properly inserted along the tool trajectory and no skiving (or patient movement) has occurred. On the other hand, if the second image has a different center than the first image, then either the surgical tool moved away from the predetermined trajectory (e.g., due to skiving), the patient moved, or both. Regardless of which movement occurred, a second image with a center different than the first image evidences that the surgical tool is not properly positioned (presumably due to skiving, but possibly due to patient movement or another factor), and that the pose of the surgical tool needs to be corrected before being used to complete the surgical procedure.

The comparing of the second image to the first image, then, comprises determining whether the first and second images have the same center point on the bony anatomical element. In embodiments where the rotational position of the surgical tool relative to the tool trajectory is relevant to a desired clinical outcome, the comparing may also comprise determining whether the second image exhibits any rotation, relative to the first image, around a common center point.

One or more algorithm such as an image processing algorithm 120 may be used to compare the first and second images.

The method 400 also comprises confirming, based at least in part on the annotated image, proper placement of a surgical tool at the target contact location (step 420). As described above, if the comparing of the step 416 yields a determination that the second image and the first image share a common center point (and, if rotational position is relevant to clinical success, that the second image does not evidence any rotation about that common center point), then the proper placement of the surgical tool at the target contact location may be confirmed. In other words, no skiving has occurred. On the other hand, if the comparing of the step 416 yields a determination that the second image and the first image do not share a common center point (and/or, if rotational position is relevant to clinical success, that the second image exhibits rotation relative to the first image), then the proper placement of the surgical tool at the target contact location cannot be confirmed. In such instance, the step 420 may comprise generating an alert (e.g., causing a message to be displayed on a user interface such as the user interface 110, causing a light to flash, causing an alarm to sound) to alert a surgeon or other user that the surgical tool is not in the correct position (whether due to skiving or otherwise).

The present disclosure encompasses embodiments of the method 400 that comprise more or fewer steps than those described above, and/or one or more steps that are different than the steps described above.

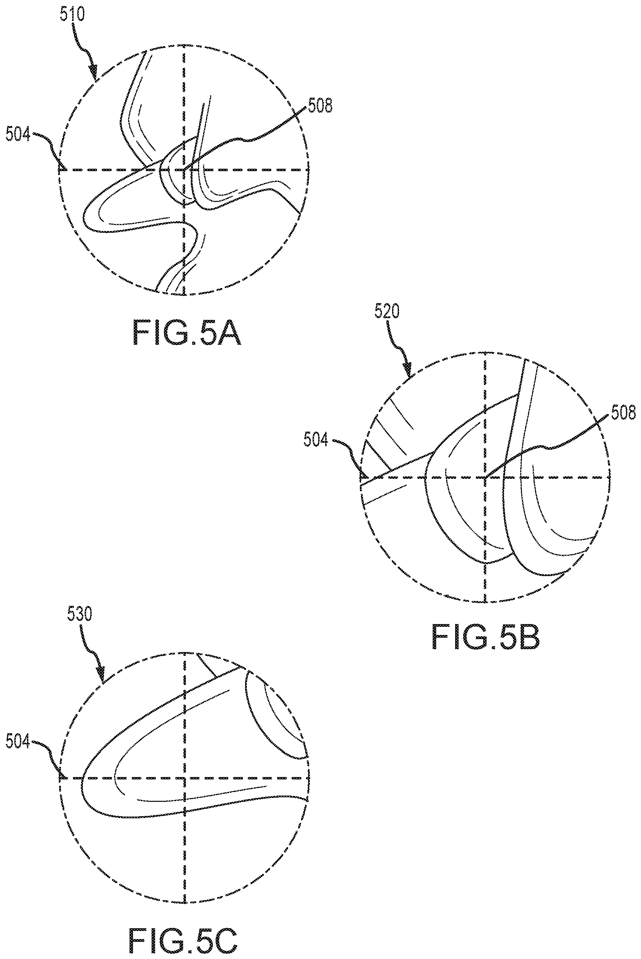

Figs. 5A-5C illustrate images that may be captured by a camera or other imaging device in connection with the method 400. More specifically, Fig. 5A depicts a sample first image 510 (such as may be captured and/or received in the step 404), which has been annotated with a digital marker 504. The digital marker 504 comprises two intersecting lines that cross at the target contact location 508.

Fig. 5B depicts a sample second image 520 (such as may be captured and/or received in the step 412). A digital marker 504 has been added to the image 520 as well. As can be seen, the second image 520 depicts a magnified or zoomed-in center portion of the image 510, with both the image 510 and the image 520 having the target contact location 508 as their center point. With the image 510 as the first image and the image 520 as the second image, the step 420 would result in confirmation of the proper placement of the surgical tool at the target contact location (e.g., a confirmation that no skiving has occurred).

Fig. 5C depicts an alternative sample second image 530 (which, again, is representative of a possible image captured and/or received in the step 412). As with the image 520, a digital marker 504 has been added to the image 530. Unlike with the image 520, however, the image 530 has a different center point than the image 510. In fact, the target contact location is not even within the field of view of the image 530. With the image 510 as the first image and the image 520 as the second image, the step 420 would not result in confirmation of the proper placement of the surgical tool at the target contact location. Instead, the step 420 may result, for example, in a surgeon or other user being alerted or notified that the surgical tool is in an incorrect pose, whether due to skiving or otherwise.

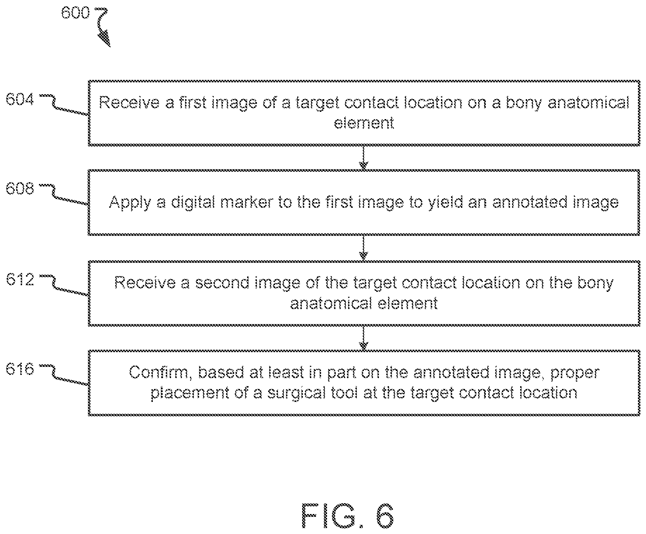

Fig. 6 depicts a method 600 that may be used, for example, to detect and avoid likely or actual skiving.

The method 600 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 600. The at least one processor may perform the method 600 by executing instructions stored in a memory such as the memory 106. The instructions may correspond to one or more steps of the method 600 described below. The instructions may cause the processor to execute one or more algorithms, such as an image processing algorithm 120, a segmentation algorithm 122, a model generation algorithm 124, and/or a skive prediction algorithm 128.

The method 600 comprises receiving a first image of a target contact location on a bony anatomical element (step 604). The first image is generated by a camera or other imaging device that is fixedly attached to a tool guide, and arm guide, or other structure that supports a surgical tool (e.g., a surgical tool 138) that will be used to contact a bony anatomical element of a patient (e.g., a drill). Unlike in the method 400, the camera or other imaging device used in the method 600 remains fixed as the surgical tool moves along the tool trajectory. However, because the camera or other imaging device is attached to a tool guide, arm guide, or other structure that supports the surgical tool, any movement of the surgical tool other than along the tool trajectory-which movement would necessarily cause the tool guide, arm guide, or other tool supporting structure to move as well-also causes movement of the camera or other imaging device.

The camera or other imaging device may be oriented parallel to the tool trajectory (e.g., where the camera or other imaging device is a borescope camera mounted through the tool guide). Alternatively, the camera or other imaging device may be oriented at an angle to the tool trajectory (e.g., where the camera or other imaging device is a borescope camera mounted through an arm guide). In either case, the camera points to the anatomical element to be contacted by the surgical tool, such that the target contact location is within a field of view of the camera or other imaging device. The camera or other imaging device may be in wired or wireless connection to a computing device (e.g., a computing device 102), a robot (e.g., a robot 114), a navigation system (e.g., a navigation system 118), or any other component of a system such as the system 100. The camera or other imaging device may be, for example, an imaging device 112.

The target contact location is a location at which the surgical tool must contact the bony anatomical element to achieve a planned result (e.g., to drill a holed in the correct pose). The bony anatomical element may a vertebra or any other bony anatomical element.

The method 600 also comprises applying a digital marker to the first image to yield an annotated image (step 608). The step 608 is the same as or similar to the step 408 of the method 400.

The method 600 also comprises receiving a second image of the target contact location on the bony anatomical element (step 612). The second image of the target contact location is captured by the same camera or other imaging device that captured the first image. However, the second image is taken after the surgical tool has been inserted into the patient. Because the camera or other imaging device is fixedly attached to a tool guide, and arm guide, or other structure that supports the surgical tool, but does not move with the surgical tool along the tool trajectory, the second image will have the same field of view as the first image if the surgical tool has moved only along the tool trajectory. If the surgical tool has moved out of the tool trajectory, however-whether due to skiving or otherwise-then the second image will have a different field of view than the first image.

The second image may, in some embodiments, be annotated to include a digital marker just as the first image.

The method 600 also comprises confirming, based at least in part on the annotated image, proper placement of a surgical tool at the target contact location (step 616). The confirming may comprise using one or more image processing algorithms such as the image processing algorithm 120 to compare the second image with the first image and determine if both images have the same field of view. If both images do have the same field of view, then proper placement of the surgical tool at the target contact location may be confirmed. If both images do not have the same field of view, then the surgical tool has moved out of the tool trajectory, and has been improperly placed. In such instance, the step 616 may comprise generating an alert (e.g., causing a message to be displayed on a user interface such as the user interface 110, causing a light to flash, causing an alarm to sound) to alert a surgeon or other user that the surgical tool is not in the correct position (whether due to skiving or otherwise).

The present disclosure encompasses embodiments of the method 600 that comprise more or fewer steps than those described above, and/or one or more steps that are different than the steps described above.

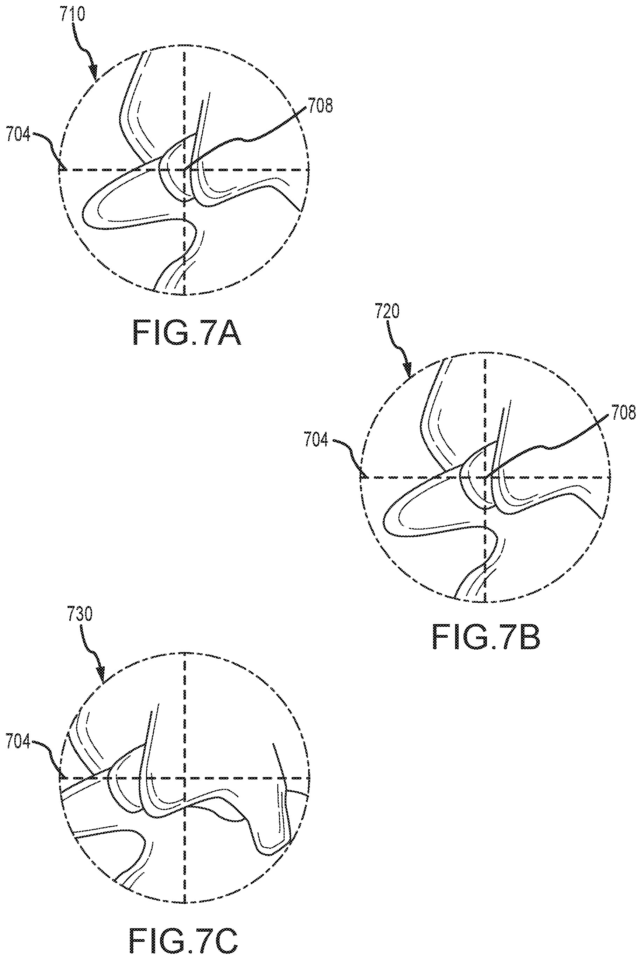

Figs. 7A-7C illustrate images that may be captured by a camera or other imaging device in connection with the method 600. More specifically, Fig. 7A depicts a sample first image 710 (such as may be captured and/or received in the step 604), which has been annotated with a digital marker 704. The digital marker 704 comprises two intersecting lines that cross at the target contact location 708.

Fig. 7B depicts a sample second image 720 (such as may be captured and/or received in the step 612). A digital marker 704 has been added to the image 720 as well. As can be seen, the second image 720 has the same field of view as the first image 710. With the image 710 as the first image and the image 720 as the second image, the step 616 would result in confirmation of the proper placement of the surgical tool (which may be, for example, a surgical tool 138) at the target contact location (e.g., a confirmation that no skiving has occurred).

Fig. 7C depicts an alternative sample second image 730 (which, again, is representative of a possible image captured and/or received in the step 612). As with the image 720, a digital marker 704 has been added to the image 730. Unlike with the image 720, however, the image 730 has a different field of view than the image 710. With the image 710 as the first image and the image 730 as the second image, the step 616 would not result in confirmation of the proper placement of the surgical tool at the target contact location. Instead, the step 616 may result, for example, in a surgeon or other user being alerted or notified that the surgical tool is in an incorrect pose, whether due to skiving or otherwise.

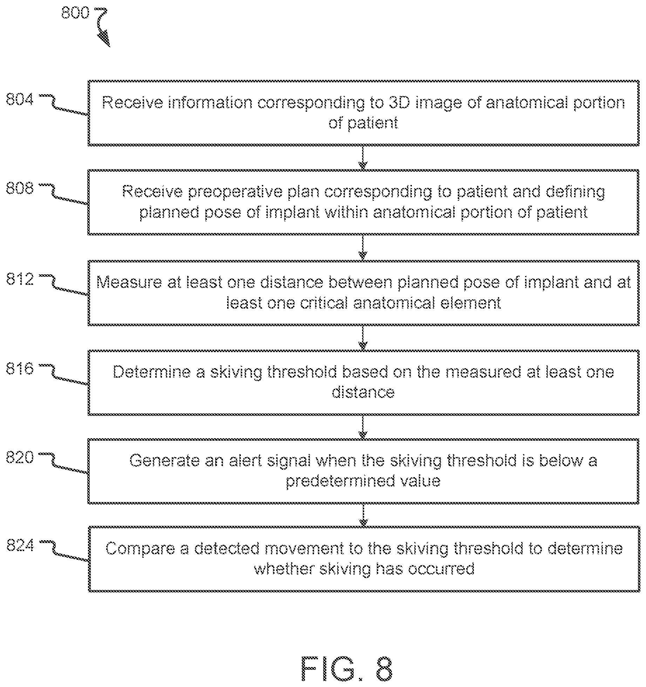

Fig. 8 depicts a method 800 that may be used, for example, to determine a skiving threshold and to detect skiving based on the skiving threshold.

The method 800 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 800. The at least one processor may perform the method 800 by executing instructions stored in a memory such as the memory 106. The instructions may correspond to one or more steps of the method 800 described below. The instructions may cause the processor to execute one or more algorithms, such as an image processing algorithm 120, a segmentation algorithm 122, a model generation algorithm 124, and/or a skive prediction algorithm 128.

The method 800 comprises receiving information corresponding to a 3D image of an anatomical portion of a patient (step 804). The 3D image may be a CT scan, an MRI scan, or a 3D image obtained using any other imaging modality. In some embodiments, the information may alternatively correspond to a plurality of 2D images of the anatomical portion of the patient, from which a 3D image or model may be generated. The anatomical portion of the patient is any anatomical portion of the patient that is relevant to a planned surgical procedure. In some embodiments, the anatomical portion of the patient is a single anatomical element that is the target of the surgical procedure, while in other embodiments, the anatomical portion of the patient includes both a target one or more anatomical elements as well as surrounding anatomy (e.g., for a spinal surgery, the anatomical portion of the patient may comprise the patient's torso). The information may be received, for example, from and/or via a memory 106, a communication interface 108, a user interface 110, an imaging device 112 (e.g., an imaging device that generated the 3D image), a database 130, and/or a network such as the cloud 134.

The method 800 also comprises receiving a preoperative plan corresponding to the patient and defining a planned pose of an implant within the anatomical portion of the patient (step 808). The preoperative plan describes some or all of the surgical procedure to be performed on one or more anatomical elements within the anatomical portion of the patient, and includes a planned pose of an implant within the anatomical portion of the patient. Thus, for example, where the preoperative plan describes the implantation of a pedicle screw within a vertebra of a patient, the preoperative plan defines a planned pose of the pedicle screw within the vertebra. In other embodiments-for example, where a hole is being drilled in a bone to gain access to another anatomical element or volume-the preoperative plane may define a planned pose for the hole.

The preoperative plan may be received, for example, from and/or via a memory 106, a communication interface 108, a user interface 110, a database 130, and/or a network such as the cloud 134.

The method 800 also comprises measuring at least one distance between the planned pose of the implant and at least one critical anatomical element (step 812). The at least one distance may be a shortest distance between any point of the planned pose of the implant and any point of the at least one critical anatomical element. The at least one distance may comprise a plurality of distances from a plurality of points of the planned pose of the implant and a plurality of points of the at least one critical anatomical element. Where the at least one critical anatomical element comprises a plurality of critical anatomical elements, the at least one distance may comprise at least one distance between the planned pose of the implant and each of the plurality of critical anatomical elements.

In some embodiments, the at least one distance may comprise a distance to a nearest critical anatomical element (if any) in each of an anterior, posterior, superior, inferior, lateral (right), and lateral (left) direction. Additionally or alternatively, the at least one distance may comprise a distance to a nearest critical anatomical element (if any) in any other direction(s).

In some embodiments, the step 812 comprises defining or otherwise determining a relative pose in space of the planned implant vis-à-vis each critical anatomical element of the at least one critical anatomical element.

Where the method 800 is being completed with respect to a planned hole rather than a planned implant, the step 812 comprises measuring at least one distance between the planned hole and at least one critical anatomical element, but otherwise is the same as described above.

As one example, when the planned implant is a pedicle screw to be implanted in a vertebra of a patient, or more broadly where a surgical procedure calls for drilling a hole in a vertebra whether for implantation of a pedicle screw, in connection with a decompression procedure, or otherwise, the critical anatomical element may be the patient's spinal cord.

The method 800 also comprises determining a skiving threshold based on the measured at least one distance (step 816). Determining the skiving threshold may comprise comparing the measured at least one distance against one or more predetermined distance thresholds or ranges, and assigning a corresponding skiving threshold. For example, a predetermined distance range of 5-10 mm may be associated with a skiving threshold of 1 mm; a predetermined distance range of 11-15 mm may be associated with a skiving threshold of 3 mm; and a predetermined distance range of 16 mm or greater may be associated with a skiving threshold of 5 mm. Using such predetermined distance ranges, if a measured distance from a planned pose of an implant to a critical anatomical element is 14 mm, then the determining may comprise determining that the measured distance of 14 mm falls within the predetermined distance range of 11-15 mm, and selecting the corresponding skiving threshold of 3 mm.

In some embodiments, the determining may be based at least in part on the length of the planned implant (or of the planned hole). For a shorter implant or hole, a given amount of tool skive in any given direction (particularly where the skive results in a change in the angle of implant or hole) will have a lesser effect than for a longer implant or hole. As a result, a determined skiving threshold may be larger for a longer implant or hole than for a smaller implant or hole.

Relatedly, the determining may also or alternatively be based at least in part on the surface contour surrounding the target contact location of the surgical tool to be used in connection with carrying out the preoperative plan. If the surface contour is flat, then skiving will impact the location of the implant or hole but may be less likely to affect the angle of the implant or hole. If the surface contour is rounded, on the other hand, then skiving may be more likely to affect both the location and the angle of the implant or hole. Thus, the likely pose of the implant or hole given skiving in any particular direction may be taken into account when determining the skiving threshold.

In some embodiments, the skiving threshold comprises a plurality of skiving thresholds. Each skiving threshold of the plurality of skiving thresholds may be direction-specific, based on a distance to a nearest critical anatomical element in the direction in question. Thus, for example, if a distance to a nearest critical anatomical element in the anterior direction is 5 mm, but a distance to a nearest critical anatomical element in the posterior direction is 20 mm, then different skiving thresholds may be determined for the anterior and posterior directions, based on the tighter tolerance needed in the anterior direction than in the posterior direction.

Also in some embodiments, determining the skiving threshold based on the measured at least one distance may comprise using one or more algorithms to calculate a skive threshold, using the measured at least one distance as an input. In some embodiments, such algorithms may receive additional inputs, such as a length of the planned implant or hole, a surface contour surrounding the target contact location, results from a Monte Carlo or other analysis regarding likely poses of an implant or hole given skiving in a particular direction and/or by a particular distance (e.g., to develop a statistical model of risk of the implant or hole contacting the critical anatomical element if skiving occurs), and/or any other useful inputs.

The method 800 also comprises generating an alert signal when the skiving threshold is below a predetermined value (step 820). Once a skiving threshold (which may comprise a plurality of skiving thresholds corresponding to a plurality of potential skiving directions) has been determined, an analysis may be conducted to determine whether a navigation system (e.g., the navigation system 118), robot (e.g., the robot 114), or other technology may be used to successfully detect skiving (e.g., to successfully detect that the surgical tool tip has slid along a contacted anatomical element a distance greater than the skiving threshold). The predetermined value may therefore be, for example, a sensitivity of the tracking technology to be used. Thus, for example, if a determined skiving threshold is 1 mm, but a navigation system that will be used to track movement of the surgical tool is only accurate to 2 mm, then an alert may be generated because the skiving threshold is below the predetermined value.

In other embodiments, the predetermined value may be set based primarily on risk tolerance, whether with or without reference to a sensitivity of the tracking technology to be used. For example, the predetermined value may be set by a surgeon, who may decide that any skiving threshold below 5 mm represents an unacceptably high risk of non-detection and of damage to a critical anatomical element. As another example, the predetermined value may be set by a navigation system or robot manufacturer.

Generating the alert may comprise, for example, causing a message to be displayed on a user interface such as the user interface 110, causing a light to flash, and/or causing an alarm to sound. The alert communicates to a surgeon or other user that the skiving threshold is too low.

The method 800 also comprises comparing a detected movement to the skiving threshold to determine whether skiving has occurred (step 824). The movement may be detected using a navigation system (e.g., the navigation system 118), a robot (e.g., the robot 114), or other technology, and may be detected in any manner described herein or in any other manner. The movement in question is movement of a tip of the surgical tool relative to the target contact location. The surgical tool may be a surgical tool 138 or any other surgical tool. Thus, if movement of a base (or any portion other than the tip) of the surgical tool is detected, then one or more calculations may be completed to determine a movement of the tip of the surgical tool. A registration between or among any two or more of a patient coordinate space, a robotic coordinate space, a navigation coordinate space, and/or any other coordinate space may be utilized in connection with such calculations. Regardless of how it is determined, the detected movement of the surgical tool tip is compared to the skiving threshold to determine if skiving has occurred. If the detected movement is less than the skiving threshold, then skiving is deemed not to have occurred. If the detected movement is greater than the skiving threshold, then skiving is deemed to have occurred. Notably, a determination that skiving has or has not occurred as described herein is a determination as to whether clinically relevant skiving has occurred. In other words, where the skiving threshold is high, the tool tip may technically have experienced skiving (e.g., have moved along a surface of an anatomical element away from the target contact location), but not to a degree that will impact a success of the procedure in question or create an unacceptable risk of damage to a critical anatomical element.

Where skiving is determined to have occurred in the step 824, the method 800 may comprise generating an alert to notify a surgeon or other user of the skiving, for example so that the surgeon or other user may correct a position of the surgical tool and/or make any other needed correction before proceeding with the surgical procedure. Generating such an alert may comprise, for example, causing a message to be displayed on a user interface such as the user interface 110, causing a light to flash, and/or causing an alarm to sound.

The present disclosure encompasses embodiments of the method 800 that comprise more or fewer steps than those described above, and/or one or more steps that are different than the steps described above.

As noted above, the present disclosure encompasses methods with fewer than all of the steps identified in Figs. 2A, 4, 6, and 8 (and the corresponding description of the methods 200, 400, 600, and 800), as well as methods that include additional steps beyond those identified in Figs. 2A, 4, 6, and 8 (and the corresponding description of the methods 200, 400, 600, and 800). The present disclosure also encompasses methods that comprise one or more steps from one method described herein, and one or more steps from another method described herein. Any correlation described herein may be or comprise a registration or any other correlation.

## Claims

1. A system (100) for skive avoidance, wherein skiving refers to an undesired slippage of a surgical tool (138) out of a target location during surgery, comprising:
a sensor (142) configured to measure a force exerted on the surgical tool (138);
at least one processor (104); and
a memory (106) storing instructions for execution by the at least one processor (104) that, when executed, cause the at least one processor (104) to:
project a tool trajectory onto a three-dimensional (3D) model of bone tissue; and
estimate an expected normal force direction and magnitude upon contact of the surgical tool (138) with the bone tissue, **characterised in that** the instructions, when executed, further cause the processor to predict a skive probability based on the expected normal force direction and magnitude.

2. The system (100) of claim 1, wherein the memory (106) stores additional instructions for execution by the at least one processor (104) that, when executed, cause the at least one processor (104) to:
receive information from the sensor (142) corresponding to a detected normal force magnitude in the expected force direction;
compare the detected normal force magnitude to the expected normal force magnitude; and
generate an alert when the detected normal force magnitude exceeds the expected normal force magnitude.

3. The system (100) of claim 1, wherein the memory (106) stores additional instructions for execution by the at least one processor (104) that, when executed, cause the at least one processor (104) to:
receive a CT scan; and
generate the 3D model based on the CT scan.

4. The system (100) of claim 3, wherein predicting the skive probability is further based on information about the surgical tool (138).

5. The system (100) of claim 1, wherein the sensor (142) is configured to measure the force exerted on the surgical tool (138) by measuring at least one force exerted on a robotic arm (116) supporting the surgical tool (138).

6. The system (100) of claim 1, wherein the memory (104) stores additional instructions for execution by the at least one processor (106) that, when executed, cause the at least one processor (106) to:
generate a torque measurement based on information about a length of the surgical tool (138) and detected force information received from the sensor (142).

## Patentansprüche

1. System (100) für eine Vermeidung von Abschälungen, wobei sich Abschälen auf ein unerwünschtes Herausrutschen eines chirurgischen Werkzeugs (138) aus einer Zielposition während einer Operation bezieht, umfassend:
einen Sensor (142), der konfiguriert ist, um eine auf das chirurgische Werkzeug (138) ausgeübte Kraft zu messen;
mindestens einen Prozessor (104); und
einen Speicher (106), der Anweisungen für eine Ausführung durch den mindestens einen Prozessor (104) speichert, die, wenn sie ausgeführt werden, den mindestens einen Prozessor (104) veranlassen zum:
Projizieren einer Werkzeugbahn auf ein dreidimensionales (3D) Modell von Knochengewebe; und
Schätzen einer erwarteten Normalkraftrichtung und -größe beim Kontakt des chirurgischen Werkzeugs (138) mit dem Knochengewebe,
**dadurch gekennzeichnet, dass** die Anweisungen, wenn sie ausgeführt werden, den Prozessor ferner veranlassen zum
Vorhersagen einer Abschälwahrscheinlichkeit basierend auf der erwarteten Normalkraftrichtung und -größe vorherzusagen.

2. System (100) nach Anspruch 1, wobei der Speicher (106) zusätzliche Anweisungen für die Ausführung durch den mindestens einen Prozessor (104) speichert, die, wenn sie ausgeführt werden, den mindestens einen Prozessor (104) veranlassen zum:
Empfangen von Informationen von dem Sensor (142), die einer erkannten Normalkraftgröße in der erwarteten Kraftrichtung entsprechen;
Vergleichen der erkannten Normalkraftgröße mit der erwarteten Normalkraftgröße; und
Erzeugen einer Warnung, wenn die erkannte Normalkraftgröße die erwartete Normalkraftgröße überschreitet.

3. System (100) nach Anspruch 1, wobei der Speicher (106) zusätzliche Anweisungen für die Ausführung durch den mindestens einen Prozessor (104) speichert, die, wenn sie ausgeführt werden, den mindestens einen Prozessor (104) veranlassen zum:
Empfangen eines CT-Scans; und
Erzeugen des 3D-Modells basierend auf dem CT-Scan.

4. System (100) nach Anspruch 3, wobei ein Vorhersagen der Abschälwahrscheinlichkeit ferner auf Informationen über das chirurgische Werkzeug (138) basiert.

5. System (100) nach Anspruch 1, wobei der Sensor (142) konfiguriert ist, um die auf das chirurgische Werkzeug (138) ausgeübte Kraft zu messen, durch Messen mindestens einer Kraft, die auf einen Roboterarm (116) ausgeübt wird, der das chirurgische Werkzeug (138) trägt.

6. System (100) nach Anspruch 1, wobei der Speicher (104) zusätzliche Anweisungen für die Ausführung durch den mindestens einen Prozessor (106) speichert, die, wenn sie ausgeführt werden, den mindestens einen Prozessor (106) veranlassen zum:
Erzeugen einer Drehmomentmessung basierend auf Informationen über eine Länge des chirurgischen Werkzeugs (138) und erkannten Kraftinformationen, die von dem Sensor (142) empfangen werden.

## Revendications

1. Système (100) d'évitement de dérapage, dans lequel le dérapage se réfère à un glissement indésirable d'un outil chirurgical (138) hors d'un emplacement cible au cours d'une intervention chirurgicale, comprenant :
un capteur (142) configuré pour mesurer une force exercée sur l'outil chirurgical (138) ;
au moins un processeur (104) ; et
une mémoire (106) stockant des instructions destinées à être exécutées par l'au moins un processeur (104) qui, lorsqu'elles sont exécutées, amènent l'au moins un processeur (104) à :
projeter une trajectoire d'outil sur un modèle tridimensionnel (3D) de tissu osseux ; et
estimer la direction et l'ampleur de la force normale attendue lors du contact de l'outil chirurgical (138) avec le tissu osseux, **caractérisé en ce que** les instructions, lorsqu'elles sont exécutées, amènent en outre le processeur à prédire une probabilité de dérapage sur la base de la direction et de l'ampleur de force normale attendue.

2. Système (100) selon la revendication 1, dans lequel la mémoire (106) stocke des instructions supplémentaires destinées à être exécutées par l'au moins un processeur (104) qui, lorsqu'elles sont exécutées, amènent en outre l'au moins un processeur (104) à :
recevoir à partir du capteur (142) des informations correspondant à une amplitude de force normale détectée dans la direction de la force attendue ;
comparer l'amplitude de force normale détectée à l'amplitude de force normale attendue ; et
générer une alerte lorsque l'amplitude de force normale détectée dépasse l'amplitude de force normale attendue.

3. Système (100) selon la revendication 1, dans lequel la mémoire (106) stocke des instructions supplémentaires destinées à être exécutées par l'au moins un processeur (104) qui, lorsqu'elles sont exécutées, amènent en outre l'au moins un processeur (104) à :
recevoir une tomodensitométrie ; et
générer le modèle 3D sur la base de la tomodensitométrie.

4. Système (100) selon la revendication 3, dans lequel la prédiction de la probabilité de dérapage est en outre sur la base des informations concernant l'outil chirurgical (138).

5. Système (100) selon la revendication 1, dans lequel le capteur (142) est configuré pour mesurer la force exercée sur l'outil chirurgical (138) en mesurant au moins une force exercée sur un bras robotique (116) supportant l'outil chirurgical (138).

6. Système (100) selon la revendication 1, dans lequel la mémoire (104) stocke des instructions supplémentaires destinées à être exécutées par l'au moins un processeur (106) qui, lorsqu'elles sont exécutées, amènent en outre l'au moins un processeur (106) à :
générer une mesure de couple sur la base des informations relatives à la longueur de l'outil chirurgical (138) et des informations de force détectée reçues à partir du capteur (142).
